# DEMANDE DE BREVET EUROPEEN

(11) **EP 4 342 508 A1**
(43) Date de publication de la demande: **27.03.2024**
(21) Numéro de dépôt: 22196981.9
(22) Date de dépôt: 21.09.2022
(51) Int. Cl.: A61M 5/178, G21F 5/018, A61M 5/145

(54) **DISPOSITIF D'ACCOUPLEMENT D'UNE SERINGUE À UN ACTIONNEUR**

(71) Demandeur: Trasis S.A., 4430 Ans (BE)
(72) Inventeur: MORELLE, Jean-Luc, 4000 LIEGE (BE); DUMONT, Philippe, 4020 JUPILLE SUR MEUSE (BE); BEKAERT, Philippe, 4500 HUY (BE)
(74) Mandataire: AWA Benelux

(57) **Abrégé**

La présente invention se rapporte, dans une forme d'exécution, à un dispositif (4) pour l'accouplement réversible d'une seringue (5) à un actionneur (3), comprenant un corps interne (41) d'axe longitudinal et un manchon externe (46) apte à se déplacer par rapport au corps interne (41) selon l'axe longitudinal, le corps interne (41) comprenant un axe coulissant terminé par un plateau de reprise de jeu (42) maintenu longitudinalement à l'intérieur du corps interne (41) en une position de repos par un ressort précontraint (43) ainsi qu'au moins une paire de crochets (44) disposés longitudinalement à l'intérieur du corps interne (41), montés pivotants et rétractables deux à deux autour de deux axes respectifs (47) perpendiculaires à l'axe longitudinal, une partie externe des crochets (44) et une partie interne du manchon (46), ladite partie externe se trouvant en vis-à-vis de ladite partie interne, ayant des profils respectifs (50, 51) configurés de sorte qu'un coulissement du manchon (46) par rapport au corps interne (41), en s'écartant des zones d'accrochage des crochets (44), provoque suite au contact desdits profils (50, 51) une ouverture des crochets (44) par pivotement autour de leurs axes de pivotement respectifs (47).

## Description

### Objet de l'invention

La présente invention se rapporte au domaine technique des dispositifs automatisés pour l'administration de doses précises de médicaments par exemple par voie intraveineuse, sous-cutanée, etc., à un patient, en particulier de doses de produits radio-pharmaceutiques.

En particulier, l'invention se rapporte à un système d'accouplement d'une seringue à un actionneur.

### Etat de la technique et position du problème

On connaît les dispositifs automatisés pour l'administration de doses radio-pharmaceutiques, qui à partir d'un flacon multidose prélèvent un volume de solution correspondant à une quantité de radioactivité demandée. Le dispositif repousse ensuite le volume prélevé dans une ligne de transfert connectée au patient. L'assemblage des lignes et autres dispositifs pour l'administration de doses au patient constituent une trousse qui doit être remplacée quotidiennement.

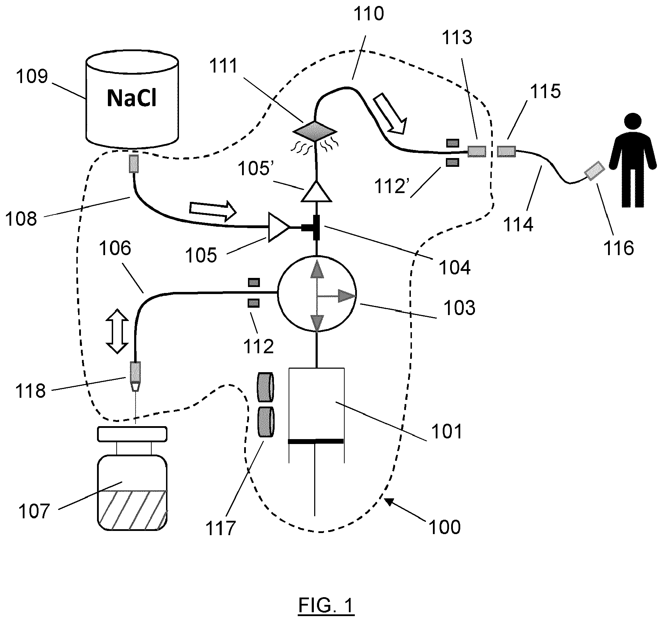

Dans un mode particulier d'exécution où le dosage est réalisé au moyen d'une seringue combinée à une vanne trois voies, une représentation d'un exemple de trousse de ce type est donnée sur le schéma de la figure 1. La trousse 100 comprend :
- une seringue 101 dont la contenance nominale est par exemple de 10mL ;
- un flacon 107 de produit radio-pharmaceutique, par exemple de contenance maximale 20mL, rempli typiquement avec 10-15mL de solution, et muni d'un septum à percer ;
- une vanne trois voies 103 ;
- un récipient contenant de la saline 109 et une ligne 108 ;
- une ligne 106 de prélèvement connectant le flacon de produit radio-pharmaceutique 107, via une aiguille 118 perçant le septum du flacon et un tube passant par un détecteur de bulles 112,à la vanne trois voies 103 ;
- un « T » 104 reliant la vanne trois voies 103 à la ligne 108 de solution NaCl, à la ligne d'injection 110 et à la seringue 101
- la ligne 108 connectant le récipient de saline 109 à la ligne d'injection 110 en passant par une vanne anti-retour 105 ;
- la ligne d'injection 110 comprenant un vanne anti-retour 105', un filtre ventilé 111 et un raccord Luer auto-obturant 113. Cette ligne traverse un détecteur de bulles 112' en amont du raccord auto-obturant 113 ;
- un prolongateur patient 114 connecté en aval de la ligne 110 grâce à un raccord Luer 115 et comprenant à son autre extrémité un autre raccord Luer 116, le connecteur 116 se connectant soit à un cathéter, soit à une ligne de transfusion, soit encore à une autre seringue pour la préparation d'une dose dans une seringue externe à la machine en vue de l'administration manuelle de cette dose à un patient ;
- un activimètre 117 se trouvant à hauteur de la seringue 101.

La séquence automatisée des opérations réalisées au moyen de la trousse 100 est la suivante :
- une certaine quantité de solution est prélevée du flacon 107 par aspiration au travers la ligne de prélèvement 106 et via la vanne trois voies 103 par la seringue 101 dont le piston est retiré par l'actionneur, la voie de prélèvement étant ouverte et la voie d'injection fermée par la vanne trois voies 103 ;
- la concentration de la solution injectable peut être ajustée en prélevant via la vanne trois voies une quantité appropriée de saline dans le récipient de saline 109, via la ligne 108, qui est aspirée dans la seringue 101 ;
- la solution éventuellement diluée est injectée via la vanne trois voies 103 par la ligne d'injection 110, la voie de prélèvement 106 étant fermée. Le filtre ventilé 111 empêche le passage de bulles vers le patient et le détecteur 112' le vérifie ;
- la solution est acheminée vers le prolongateur patient 114.

L'homme de métier qui est amené à concevoir un tel dispositif doit tenir compte de diverses considérations qui déterminent :
- la précision requise pour le prélèvement : la dose administrée au patient doit être très précise (par ex. +/-5%), quelle que soit la concentration du produit de départ. Tant la concentration du produit de départ que la dose à administrer (qui est dépendante du poids du patient, peut même être pédiatrique) sont très variables ;
- le choix du volume maximal de la seringue et
- la maitrise de la pression exercée sur la seringue lors de son actionnement.

D'autres considérations d'ordre ergonomique et pratique déterminent les opérations de placement et de remplacement de la trousse incluant la seringue.

Selon ces considérations, le placement et le remplacement de la trousse sur son dispositif d'actionnement, appelé injecteur, doit être simple et rapide. Le fonctionnement et la performance du système d'administration doivent être indépendants de la dextérité de l'opérateur.

En particulier l'arrimage et le désarrimage du poussoir de la seringue à l'accouplement de l'actionneur, une fois la trousse mise en place, doit être automatique. Cet accouplement ne doit inclure aucun composant « actif », c'est à dire qui nécessiterait d'être spécifiquement activé par le système de contrôle au-delà de l'actionneur de seringue lui-même. Ce mécanisme d'accrochage et de libération du poussoir doit donc être passif. En outre, lorsque le poussoir est arrimé à l'accouplement de l'actionneur, aucun jeu ne peut subsister entre celui-ci et l'actionneur.

### Buts de l'invention

La présente invention vise à fournir un dispositif d'accouplement simple, précis et rapide entre des trousses radiopharmaceutiques et un automate de distribution de dose à un patient.

L'invention vise également à produire un accouplement sans jeu de l'actionneur avec le poussoir de la seringue.

### Principaux éléments caractéristiques de l'invention

Un premier aspect de la présente invention se rapporte à un dispositif pour l'accouplement réversible d'une seringue à un actionneur, comprenant un corps interne d'axe longitudinal et un manchon externe apte à se déplacer par rapport au corps interne selon l'axe longitudinal, le corps interne comprenant un axe coulissant terminé par un plateau de reprise de jeu maintenu longitudinalement à l'intérieur du corps interne en une position de repos par un ressort précontraint ainsi qu'au moins une paire de crochets disposés longitudinalement à l'intérieur du corps interne, montés pivotants et rétractables deux à deux autour de deux axes respectifs perpendiculaires à l'axe longitudinal, une partie externe des crochets et une partie interne du manchon, ladite partie externe se trouvant en vis-à-vis de ladite partie interne, ayant des profils respectifs configurés de sorte qu'un coulissement du manchon par rapport au corps interne, en s'écartant des zones d'accrochage des crochets, provoque suite au contact desdits profils une ouverture des crochets par pivotement autour de leurs axes de pivotement respectifs.

Selon des formes d'exécution préférées, le dispositif comporte en outre au moins une des caractéristiques suivantes, ou une combinaison appropriée de plusieurs d'entre elles :
- les crochets d'une même paire possèdent chacun une zone d'accrochage à une extrémité proximale du plateau de reprise de jeu, et sont connectés l'un à l'autre à une extrémité distale par un ressort de rappel, la partie distale des crochets étant pourvue, vers l'extérieur, d'un bossage apte à s'insérer dans une zone creuse d'une première profondeur du profil interne du manchon dans une position de repos du dispositif d'accouplement où les crochets sont redressés et en position de blocage du bouton du poussoir, le coulissement relatif du manchon par rapport au corps interne, dans lequel le manchon s'écarte de ladite extrémité distale des crochets, provoquant l'ouverture des crochets par pivotement autour de leurs axes de pivotement respectifs, du fait que le bossage de chaque crochet entre, vers le bas, dans une zone creuse d'une seconde profondeur du profil interne du manchon, la zone creuse d'une seconde profondeur étant adjacente à la zone creuse d'une première profondeur, la première profondeur étant supérieure à la seconde profondeur ;
- le dispositif comporte de 2 à 4 paires de crochets ;
- les crochets possèdent une région d'accrochage définie par une partie saillante avec un chanfrein en pente vers l'intérieur, suivie d'un renfoncement, en dessous de la parie saillante, le renfoncement étant dimensionné pour bloquer sans jeu, au moyen des crochets, le plateau de reprise de jeu poussé par son ressort précontraint et accolé à un bouton de poussoir d'une seringue.

Un deuxième aspect de l'invention se rapporte à un accouplement réversible d'une seringue pour l'injection de produit dosé et d'un actionneur, ladite seringue étant munie d'un poussoir avec un bouton de poussoir, ledit actionneur étant apte à être accouplé au poussoir de la seringue au moyen du dispositif décrit ci-dessus, caractérisé en ce que, en position accouplée, le bouton du poussoir de la seringue est bloqué dans le renfoncement entre la partie saillante des crochets (44) et le plateau de reprise de jeu qui, grâce au ressort précontraint, exerce une force dirigée vers le poussoir, de sorte à éliminer tout jeu entre le poussoir et le dispositif d'accouplement.

Avantageusement, l'accouplement réversible comporte un capteur de force disposé derrière le mécanisme d'accouplement entre l'actionneur et le dispositif d'accouplement et apte à mesurer directement les efforts pendant l'injection et pendant l'accouplement.

Un troisième aspect de l'invention se rapporte à une machine automatique d'administration d'une dose de produit, comprenant un actionneur (3) et au moins une seringue (5) pour l'injection de produit dosé munie d'un poussoir (6) avec un bouton de poussoir (7), et un accouplement réversible de la seringue (5) et de l'actionneur (3), selon la revendication 5.

De préférence, la machine automatique d'administration d'une dose de produit est une machine pour administrer un médicament radiopharmaceutique à un patient et la machine comprend également une trousse jetable radiopharmaceutique comprenant au moins une seringue pour l'administration de la dose au patient.

Un quatrième aspect de l'invention se rapporte à une méthode d'accouplement réversible d'au moins une seringue pour l'injection d'un produit dosé et d'un actionneur, mettant en oeuvre le dispositif d'accouplement décrit ci-dessus, caractérisée par les étapes suivantes :
- le dispositif d'accouplement est en position de repos, avec le corps faisant saillie vers le haut quand la seringue se trouve au-dessus du dispositif d'accouplement, respectivement vers le bas quand la seringue se trouve en dessous du dispositif d'accouplement, et relativement au manchon en direction de la seringue ;
- l'actionneur pousse l'accouplement vers le bouton du poussoir de la seringue ;
- le bouton du poussoir pousse sur les crochets au niveau de leur chanfrein et provoque l'ouverture des crochets, les bossages des crochets passant successivement d'un contact avec la zone creuse d'une première profondeur du profil interne du manchon à une position sans contact ;
- dès que le bouton du poussoir a atteint l'extrémité de la partie saillante des crochets, la fermeture des crochets sur le bouton du poussoir est automatique, grâce au ressort de rappel, le bouton du poussoir et le plateau de reprise de jeu entrant en contact et s'insérant dans le renfoncement ; et
- le bouton du poussoir est bloqué sans jeu entre les crochets et le plateau de reprise de jeu.

Un cinquième aspect de l'invention se rapporte à une méthode de désolidarisation réversible d'une seringue pour l'injection d'un produit dosé et d'un actionneur, mettant en œuvre le dispositif d'accouplement décrit ci-dessus, caractérisée par les étapes suivantes :
- à partir de la position de repos de l'accouplement, l'actionneur tire celui-ci vers le bas, respectivement vers le haut, selon la position relative de l'actionneur et de la seringue, provoquant un déplacement de retrait du poussoir dans la seringue ;
- le manchon arrive à butée sur une surface d'appui du châssis de l'actionneur et s'immobilise alors que le corps continue à descendre, respectivement à monter, sous l'effet de l'actionneur ;
- le profil extérieur des crochets coopère par contact avec le profil intérieur creux du manchon, les bossages des crochets passant successivement d'un contact avec la zone creuse d'une première profondeur du profil interne du manchon à un contact avec la zone creuse d'une seconde profondeur dudit profil, de manière à faire pivoter et ouvrir les crochets ;
- le bouton du poussoir se désolidarise de l'accouplement et, si une dépression a été créée au préalable dans la seringue par fermeture d'une vanne communiquant dans une ligne en aval avec la seringue, le poussoir remonte automatiquement dans le corps de la seringue dès l'ouverture des crochets ;
- la seringue est libérée de l'accouplement et peut être retirée.

Alternativement, la méthode de désolidarisation réversible d'une seringue pour l'injection d'un produit dosé et d'un actionneur, mettant en œuvre le dispositif décrit ci-dessus, est caractérisée par les étapes suivantes :
- à partir de la position de repos de l'accouplement, on soulève, respectivement on abaisse, manuellement le manchon en direction du poussoir de la seringue ;
- le profil extérieur des crochets coopère par contact avec le profil intérieur creux du manchon de manière à faire pivoter et ouvrir les crochets, les bossages des crochets passant successivement d'un contact avec la zone creuse d'une première profondeur du profil interne du manchon à un contact avec la zone creuse d'une seconde profondeur dudit profil ;
- le bouton du poussoir se désolidarise de l'accouplement et, si une dépression a été créée au préalable dans la seringue par fermeture d'une vanne communiquant dans une ligne en aval avec la seringue, le poussoir remonte, respectivement descend, automatiquement dans le corps de la seringue dès l'ouverture des crochets, un retrait de l'actionneur permettant alternativement de dégager le bouton du poussoir du dispositif d'accouplement ;
- la seringue est libérée de l'accouplement et peut être retirée, soit utilisée en place pour poursuivre des opérations de prélèvement et d'injection par des mouvements manuels du poussoir.

Ainsi, en cas de panne de la machine, le fonctionnement manuel permet de dégager le poussoir de l'actionneur défectueux.

### Brève description des figures

La figure 1 montre schématiquement les composants et le fonctionnement d'une trousse utilisée dans un dispositif automatique d'administration de dose radio-pharmaceutique dans un mode particulier d'exécution de l'invention.
La figure 2 représente une vue globale tridimensionnelle de l'actionneur, de la seringue et du dispositif d'accouplement des deux éléments selon une forme d'exécution de la présente invention.
La figure 3 représente différentes vues en coupe de l'accouplement et de ses composants (selon des plans orthogonaux).
La figure 4 représente des vues de l'accouplement respectivement en position avec crochets fermés (a) et crochets ouverts (b).
La figure 5 représente en plusieurs vues le mécanisme de solidarisation de la seringue avec mouvement relatif de l'accouplement vis-à-vis de la seringue et ouverture/fermeture des crochets.
La figure 6 représente en plusieurs vues le mécanisme de désolidarisation de la seringue avec mouvement relatif de l'accouplement vis-à-vis de la seringue et ouverture/fermeture des crochets.

### Description détaillée de l'invention

Un système spécifique de préhension du poussoir de la seringue a été développé selon la présente invention.

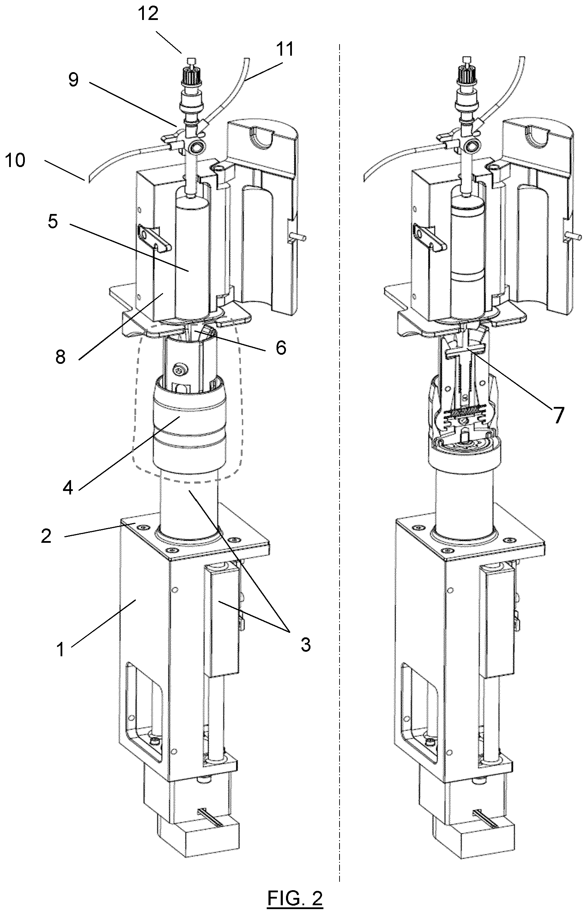

Sur la figure 2 est représentée une structure fixe 1 avec sa surface d'appui 2 pour l'accouplement, l'actionneur 3 de seringue, son dispositif d'accouplement 4, la seringue avec son corps 5 et son poussoir 6 en position solidarisée à l'accouplement 4, le système de fixation 8 de la seringue sur un châssis, la vanne trois-voies 9 et le départ des tubes respectifs de prélèvement 10, de connexion de saline 11 et d'injection 12.

Sur la partie droite de la figure 2, le détail de l'accouplement 4 est montré selon une vue en coupe. On voit également le bouton du poussoir 7 de la seringue inséré dans l'accouplement.

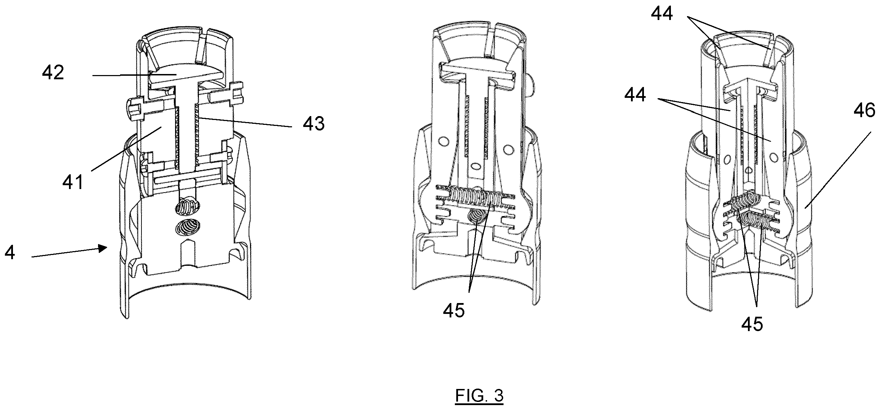

La figure 3 représente le dispositif d'accouplement passif 4 seul avec ses différents composants, à savoir :
- un corps de l'accouplement 41 ;
- un plateau de reprise du jeu 42 et son ressort précontraint 43 ;
- plusieurs crochets pivotants 44 ;
- un ressort de rappel précontraint 45 pour chaque paire de crochets 44 ; et
- un manchon 46 de contrôle d'ouverture des crochets 44.

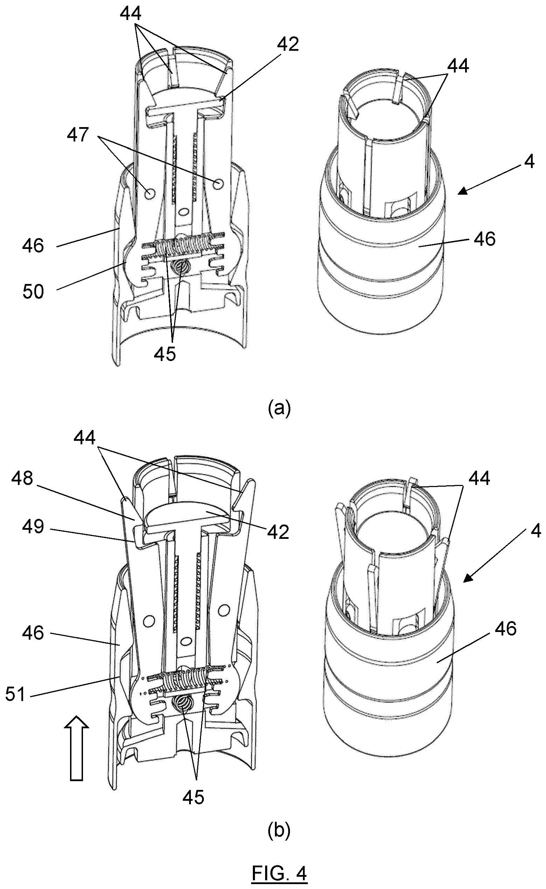

La figure 4a) montre l'accouplement 4 dans son état par défaut (ou de repos), à savoir crochets 44 fermés. La figure 4b) montre l'accouplement 4 avec ses crochets 44 ouverts suite au mouvement relatif du manchon 46 par rapport au corps de l'accouplement 41.

Selon une forme d'exécution, les crochets 44 sont montés longitudinalement par paires, les crochets d'une même paire étant reliés par un ressort de rappel 45 disposé selon un axe perpendiculaire à l'axe moyen des crochets et solidarisé aux crochets dans leur partie inférieure. Il y a par exemple deux paires perpendiculaires 44 de crochets, leurs ressorts respectifs 45 étant également perpendiculaires et situés à des hauteurs différentes. Les crochets 44 sont montés pivotants autour d'un axe 47 se trouvant plus ou moins à mi-hauteur du crochet. Dans le sens de haut en bas, chaque crochet 44 possède une partie saillante vers l'intérieur 48 suivie d'un renfoncement 49, ce qui va permettre d'exercer la fonction de blocage du bouton du poussoir 7 de la seringue contre le plateau de reprise de jeu 42, une fois joints. Par ailleurs, le mouvement montant du manchon 46 par rapport au corps de l'accouplement 41 va permettre, selon l'invention, d'ouvrir les crochets et de libérer le bouton du poussoir 7 et le poussoir 6 de la seringue. A cette fin, la structure externe des crochets 44 doit coopérer avec la structure interne du manchon 46. Selon une forme d'exécution, la partie inférieure externe des crochets est munie d'un bossage 50 et la partie interne du manchon est munie d'un profil creux 51 correspondant au déplacement souhaité du bossage 50 lorsque le manchon monte relativement au corps de l'accouplement. Dans la position par défaut, les crochets 44 de l'accouplement 4 étant fermés, le bossage 50 des crochets se trouve dans le creux du profil 51 du manchon (figure 4a)). Lorsque le corps d'accouplement 41 va descendre relativement au manchon, le bossage 50 va sortir vers le bas du profil intérieur du manchon 51 et le contact du bossage 50 avec le bord inférieur du profil 51 qui fait saillie va provoquer automatiquement le pivotement de chaque crochet 44 autour de son axe de pivotement 47, de sorte que le crochet s'ouvre vers l'extérieur et libère le bouton du poussoir 7 et par conséquent le poussoir 6 de la seringue.

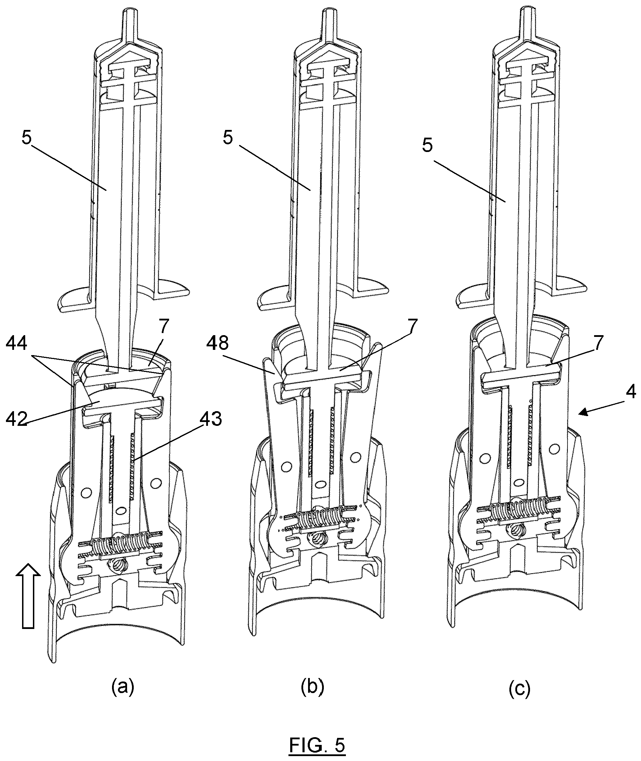

La figure 5 représente en trois étapes successives le mécanisme de solidarisation de la seringue 5 (supposée fixe) avec mouvement relatif de l'accouplement 4 vis-à-vis de la seringue 5 et ouverture/fermeture des crochets 44.

En se déplaçant dans le sens de l'injection, l'accouplement 4 accoste le bouton du poussoir 7 de la seringue 5 et s'y accroche. L'ouverture des crochets 44 est provoquée par le contact du bouton du poussoir 7 de la seringue 5 sur les crochets 44 (figure 5b). La fermeture des crochets 44 sur le bouton du poussoir 7 se produit dès que le bouton du poussoir 7 de la seringue 5 dépasse l'extrémité des parties saillantes 48 des crochets 24 (figure 5b) en comprimant ce faisant le plateau de reprise de jeu 42 monté sur le ressort précontraint 43 pour venir s'encastrer dans le renfoncement 49 des crochets (figure 5c).

La plateau 42 monté sur son ressort précontraint 43 reprend les jeux entre les crochets de préhension 44 et la surface d'appui supérieure du bouton du poussoir 7 et entre la surface d'appui inférieure du bouton du poussoir 7 et le plateau de reprise de jeu 42. Le processus d'accrochage nécessite de vaincre la force de ce ressort.

Une fois accrochés l'un à l'autre, le poussoir 6 et l'actionneur 3 sont strictement solidaires quel que soit le sens du mouvement de l'actionneur 3 (figure 5c) dans la limite de la force du ressort précontraint du plateau.

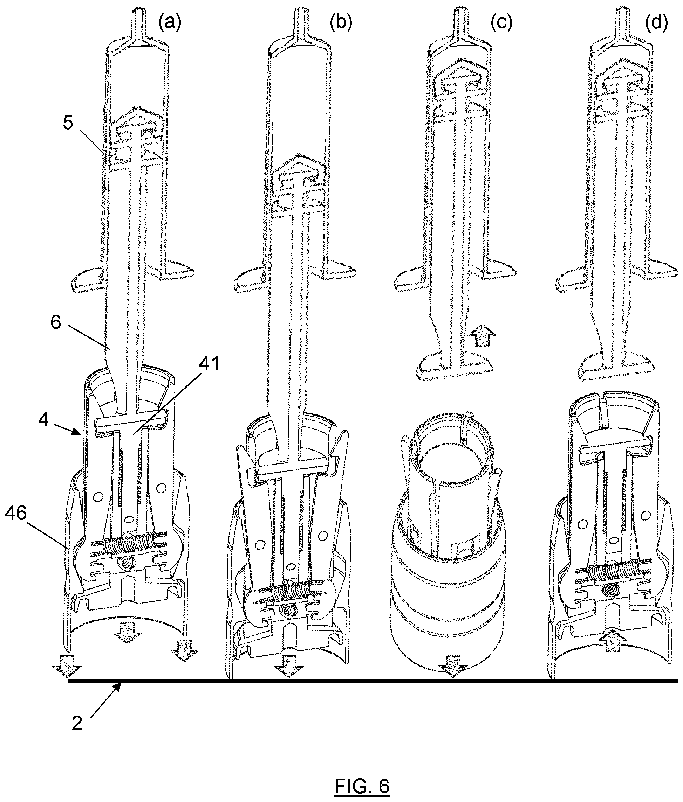

La figure 6 montre ensuite les différentes étapes de désolidarisation de la seringue (de gauche à droite). La désolidarisation s'actionne par un déplacement de retrait maximal vers le bas de l'actionneur (figure 6a). Lors de la descente de l'accouplement 4, le manchon 46 arrive en contact avec la surface d'appui 2 de la structure fixe 1 qui empêche le manchon 46 de descendre davantage, tandis que le corps 41 de l'accouplement 4 continue à descendre. Par son mouvement relatif vis-à-vis du corps de l'accouplement 41, le manchon 46 utilise son profil intérieur pour s'appuyer sur les bossages des crochets 44 de manière à les faire pivoter et les ouvrir (comme décrit ci-dessus). Les crochets s'écartant, le bouton du poussoir 7 se désolidarise alors de l'accouplement 4 et le poussoir 6 peut remonter automatiquement. En effet, si le déplacement du piston 6 vers le bas a été réalisé avec la vanne trois voies en position fermée, cela cause une dépression dans la seringue et le poussoir 6 remonte dans le corps 5 de la seringue instantanément dès l'ouverture des crochets 44. La seringue 5 est libérée de l'accouplement 4 et la trousse peut être retirée aisément de la machine.

Le désaccouplement peut également être effectué à la main en soulevant le manchon 46, l'actionneur étant à l'arrêt.

Le manchon 46 peut être solidaire soit (a) de l'accouplement (comme montré ci-dessus), soit (b) de la surface d'appui de la structure fixe. Notons que, dans ce dernier cas, il ne pourrait pas être actionné manuellement.

### Description de formes d'exécution préférées de l'invention

Dans une forme préférée d'exécution de l'invention, la seringue est de contenance nominale de 10mL pour une course de l'ordre de 48 mm, avec pour le piston la possibilité de se déplacer de plusieurs mm au-delà du remplissage nominal. De 10 à 15mL représente un volume habituel de substance radiopharmaceutique livrée en flacon pour la TEP (Tomographie par Emission de Positrons).

Une dose pédiatrique de 18F-FDG par exemple peut être aussi petite que 30MBq, tandis qu'une dose adulte est de l'ordre de 200 à 300MBq. Si la concentration de la solution de départ est élevé, par exemple de 30GBq dans 10 mL, et que l'on veut préparer une dose pédiatrique, on se trouve dans le cas le plus difficile. Le volume à prélever est alors de 10*(30/30000) mL = 0.01mL (=10µL).

Le plus petit prélèvement envisagé, et donc la plus petite variation de volume (ΔV) visée est de ~0.01mL avec une précision attendue de ±10%. Ce ΔV correspond à un déplacement de 0.01[mL]*(48mm/10mL) = 0.048 mm, soit 48 ± 6 µm.

La résolution du pousse-seringue étant ~2µm, de tels dosages ne sont possibles avec la précision voulue qu'en l'absence de tout jeu et avec une bonne maîtrise des forces appliquées.

Selon une autre forme d'exécution de l'invention, un capteur de force (non représenté) est disposé derrière le mécanisme d'accouplement entre l'actionneur et le dispositif d'accouplement et permet de mesurer directement les efforts pendant l'injection et pendant l'accouplement, ce qui permet notamment :
- en faisant le vide dans la seringue, de vérifier que l'accouplement a bien été réalisé ;
- de vérifier les différences de pressions dans la trousse car la pression exercée par la substance présente dans la seringue sur le piston de la seringue est transmise au bouton du poussoir lui-même directement connecté à l'accouplement, qui est lui-même connecté à l'actionneur via le capteur de force en question. On peut ainsi détecter des anomalies comme un tube croqué dans la trousse lors d'un mouvement de fluide avec la seringue.

### Liste des symboles de référence

- 1: structure fixe (châssis)
- 2: surface d'appui
- 3: actionneur
- 4: accouplement
- 5: seringue (corps)
- 6: poussoir
- 7: bouton du poussoir
- 8: système de fixation de la seringue
- 9: vanne trois-voies
- 10: ligne de prélèvement
- 11: ligne de connexion de saline
- 12: ligne d'injection
- 41: corps d'accouplement
- 42: plateau de reprise de jeu
- 43: ressort précontraint (du plateau de reprise de jeu)
- 44: crochet pivotant
- 45: ressort de rappel (paire de crochets)
- 46: manchon d'accouplement
- 47: axe pivot
- 48: partie saillante du crochet
- 49: renfoncement du crochet
- 50: bossage
- 51: profil intérieur du manchon
- 100: trousse radiopharmaceutique
- 101: seringue
- 102: ligne de connexion vanne-seringue
- 103: vanne trois-voies
- 104: tube en « T »
- 105, 105': vanne anti-retour
- 106: ligne de prélèvement
- 107: flacon de produit radiopharmaceutique concentré
- 108: ligne de connexion de saline
- 109: flacon de saline
- 110: ligne d'injection
- 111: filtre ventilé
- 112, 112': détecteur de bulles
- 113: Luer auto-obturant
- 114: prolongateur patient
- 115: raccord Luer machine
- 116: raccord Luer patient
- 117: activimètre
- 118: aiguille de perçage

## Revendications

1. Un dispositif (4) pour l'accouplement réversible d'une seringue (5) à un actionneur (3), comprenant un corps interne (41) d'axe longitudinal et un manchon externe (46) apte à se déplacer par rapport au corps interne (41) selon l'axe longitudinal, le corps interne (41) comprenant un axe coulissant terminé par un plateau de reprise de jeu (42) maintenu longitudinalement à l'intérieur du corps interne (41) en une position de repos par un ressort précontraint (43) ainsi qu'au moins une paire de crochets (44) disposés longitudinalement à l'intérieur du corps interne (41), montés pivotants et rétractables deux à deux autour de deux axes respectifs (47) perpendiculaires à l'axe longitudinal, une partie externe des crochets (44) et une partie interne du manchon (46), ladite partie externe se trouvant en vis-à-vis de ladite partie interne, ayant des profils respectifs (50, 51) configurés de sorte qu'un coulissement du manchon (46) par rapport au corps interne (41), en s'écartant des zones d'accrochage des crochets (44), provoque suite au contact desdits profils (50, 51) une ouverture des crochets (44) par pivotement autour de leurs axes de pivotement respectifs (47).

2. Le dispositif (4) selon la revendication 1, **caractérisé en ce que** les crochets (44) d'une même paire possèdent chacun une zone d'accrochage (48, 49) à une extrémité proximale du plateau de reprise de jeu (42), et sont connectés l'un à l'autre à une extrémité distale par un ressort de rappel (45), la partie distale des crochets (44) étant pourvue, vers l'extérieur, d'un bossage (50) apte à s'insérer dans une zone creuse d'une première profondeur du profil (51) interne du manchon (46) dans une position de repos du dispositif d'accouplement (4) où les crochets (44) sont redressés et en position de blocage du bouton du poussoir (7), le coulissement relatif du manchon (46) par rapport au corps interne (41), dans lequel le manchon (46) s'écarte de ladite extrémité distale des crochets (44), provoquant l'ouverture des crochets (44) par pivotement autour de leurs axes de pivotement respectifs (47), du fait que le bossage (50) de chaque crochet (44) entre, vers le bas, dans une zone creuse d'une seconde profondeur du profil (51) interne du manchon (46), la zone creuse d'une seconde profondeur étant adjacente à la zone creuse d'une première profondeur, la première profondeur étant supérieure à la seconde profondeur.

3. Le dispositif (4) selon la revendication 1, **caractérisé en ce qu'**il comporte de 2 à 4 paires de crochets (44).

4. Le dispositif (4) selon la revendication 2, **caractérisé en ce que** les crochets (44) possèdent une région d'accrochage définie par une partie saillante (48) avec un chanfrein en pente vers l'intérieur, suivie d'un renfoncement (49), en dessous de la parie saillante (48), le renfoncement (49) étant dimensionné pour bloquer sans jeu, au moyen des crochets (44), le plateau de reprise de jeu (42) poussé par son ressort précontraint (43) et accolé à un bouton de poussoir (7) d'une seringue (5).

5. Accouplement réversible d'une seringue (5) pour l'injection de produit dosé et d'un actionneur (3), ladite seringue (5) étant munie d'un poussoir (6) avec un bouton de poussoir (7), ledit actionneur (3) étant apte à être accouplé au poussoir (6) de la seringue (5) au moyen du dispositif (4) selon l'une quelconque des revendications précédentes, **caractérisé en ce que**, en position accouplée, le bouton du poussoir (7) de la seringue (5) est bloqué dans le renfoncement (49) entre la partie saillante (48) des crochets (44) et le plateau de reprise de jeu (42) qui, grâce au ressort précontraint (43), exerce une force dirigée vers le poussoir (7), de sorte à éliminer tout jeu entre le poussoir (7) et le dispositif d'accouplement (4).

6. Accouplement réversible selon la revendication 5, **caractérisé en ce qu'**il comporte un capteur de force disposé derrière le mécanisme d'accouplement entre l'actionneur (3) et le dispositif d'accouplement (4) et apte à mesurer directement les efforts pendant l'injection et pendant l'accouplement.

7. Une machine automatique d'administration d'une dose de produit, comprenant un actionneur (3) et au moins une seringue (5) pour l'injection de produit dosé munie d'un poussoir (6) avec un bouton de poussoir (7), et un accouplement réversible de la seringue (5) et de l'actionneur (3), selon la revendication 5.

8. La machine automatique d'administration d'une dose de produit selon la revendication 7, **caractérisée en ce que** le produit est un médicament radiopharmaceutique et **en ce que** la machine comprend également une trousse jetable radiopharmaceutique (100) comprenant au moins une seringue (5) pour l'administration de la dose au patient.

9. Une méthode d'accouplement réversible d'au moins une seringue (5) pour l'injection d'un produit dosé et d'un actionneur (3), mettant en œuvre le dispositif d'accouplement (4) selon l'une quelconque des revendications 1 à 4, **caractérisée par** les étapes suivantes :
- le dispositif d'accouplement (4) est en position de repos, avec le corps (41) faisant saillie vers le haut quand la seringue se trouve au-dessus du dispositif d'accouplement (4), respectivement vers le bas quand la seringue se trouve en dessous du dispositif d'accouplement (4), et relativement au manchon (46) en direction de la seringue (5) ;
- l'actionneur pousse l'accouplement (4) vers le bouton du poussoir (7) de la seringue (5) ;
- le bouton du poussoir (7) pousse sur les crochets (44) au niveau de leur chanfrein et provoque l'ouverture des crochets (44), les bossages (50) des crochets (44) passant successivement d'un contact avec la zone creuse d'une première profondeur du profil (51) interne du manchon (46) à une position sans contact ;
- dès que le bouton du poussoir (7) a atteint l'extrémité de la partie saillante (48) des crochets (44), la fermeture des crochets (44) sur le bouton du poussoir (7) est automatique, grâce au ressort de rappel (45), le bouton du poussoir (7) et le plateau de reprise de jeu (42) entrant en contact et s'insérant dans le renfoncement (49) ; et
- le bouton du poussoir (7) est bloqué sans jeu entre les crochets (44) et le plateau de reprise de jeu (42).

10. Une méthode de désolidarisation réversible d'une seringue (5) pour l'injection d'un produit dosé et d'un actionneur (3), mettant en œuvre le dispositif d'accouplement (4) selon l'une quelconque des revendications 1 à 4, **caractérisée par** les étapes suivantes :
- à partir de la position de repos de l'accouplement (4), l'actionneur tire celui-ci vers le bas, respectivement vers le haut, selon la position relative de l'actionneur et de la seringue, provoquant un déplacement de retrait du poussoir (6) dans la seringue (5) ;
- le manchon (46) arrive à butée sur une surface d'appui (2) du châssis (1) de l'actionneur (3) et s'immobilise alors que le corps (41) continue à descendre, respectivement à monter, sous l'effet de l'actionneur (3) ;
- le profil extérieur des crochets (44) coopère par contact avec le profil intérieur creux du manchon (46), les bossages (50) des crochets (44) passant successivement d'un contact avec la zone creuse d'une première profondeur du profil (51) interne du manchon (46) à un contact avec la zone creuse d'une seconde profondeur dudit profil (51), de manière à faire pivoter et ouvrir les crochets (44) ;
- le bouton du poussoir (7) se désolidarise de l'accouplement (4) et, si une dépression a été créée au préalable dans la seringue (5) par fermeture d'une vanne communiquant dans une ligne en aval avec la seringue, le poussoir (6) remonte automatiquement dans le corps (5) de la seringue dès l'ouverture des crochets (44) ;
- la seringue (5) est libérée de l'accouplement (4) et peut être retirée.

11. Une méthode de désolidarisation réversible d'une seringue (5) pour l'injection d'un produit dosé et d'un actionneur (3), mettant en œuvre le dispositif d'accouplement (4) selon l'une quelconque des revendications 1 à 4, **caractérisée par** les étapes suivantes :
- à partir de la position de repos de l'accouplement (4), on soulève, respectivement on abaisse, manuellement le manchon (46) en direction du poussoir (6) de la seringue (5) ;
- le profil extérieur des crochets (44) coopère par contact avec le profil intérieur creux du manchon (46) de manière à faire pivoter et ouvrir les crochets (44), les bossages (50) des crochets (44) passant successivement d'un contact avec la zone creuse d'une première profondeur du profil (51) interne du manchon (46) à un contact avec la zone creuse d'une seconde profondeur dudit profil (51) ;
- le bouton du poussoir (7) se désolidarise de l'accouplement (4) et, si une dépression a été créée au préalable dans la seringue (5) par fermeture d'une vanne communiquant dans une ligne en aval avec la seringue, le poussoir (6) remonte, respectivement descend, automatiquement dans le corps (5) de la seringue dès l'ouverture des crochets (44), un retrait de l'actionneur (3) permettant alternativement de dégager le bouton du poussoir (7) du dispositif d'accouplement (4) ;
- la seringue (5) est libérée de l'accouplement (4) et peut être retirée, soit utilisée en place pour poursuivre des opérations de prélèvement et d'injection par des mouvements manuels du poussoir (7).
